(19) Europäisches Patentamt
European Patent Office
Office européen des brevets

(11) Veröffentlichungsnummer: **0 240 463 B1**

# (12) EUROPÄISCHE PATENTSCHRIFT

(45) Veröffentlichungstag der Patentschrift: **22.11.90**

(21) Anmeldenummer: **87810184.9**

(22) Anmeldetag: **30.03.87**

(51) Int. Cl.⁵: **C07C 233/31**, C07C 233/02, C07D 295/18, C08K 5/32, C08K 5/34

## (54) Substituierte Aminoxypropionamide.

(30) Priorität: **04.04.86 US 848106**

(43) Veröffentlichungstag der Anmeldung: **07.10.87 Patentblatt 87/41**

(45) Bekanntmachung des Hinweises auf die Patenterteilung: **22.11.90 Patentblatt 90/47**

(84) Benannte Vertragsstaaten: **BE CH DE FR GB IT LI NL SE**

(56) Entgegenhaltungen:
**CHEMICAL ABSTRACTS, Band 68, Nr. 21, 20. Mai 1968, Seiten 8977-8978, Zusammenfassung Nr. 93173a, Columbus, Ohio, US; L.P. SASHCHENKO et al.: "Inhibition of L-glutamate decarboxylase by derivatives of hydroxylamine and related compounds", & BIOKHIMIYA 33(1), 142-7(1968) 000**

**Die Akte enthält technische Angaben, die nach dem Eingang der Anmeldung eingereicht wurden und die nicht in dieser Patentschrift enthalten sind.**

(73) Patentinhaber: **CIBA-GEIGY AG, Klybeckstrasse 141, CH-4002 Basel(CH)**

(72) Erfinder: **Ravichandran, Ramanathan, 111 DeHaven Drive Apt. 125, Yonkers New York 10703(US)**

EP 0 240 463 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Aminoxypropionamide, deren Verwendung zum Stabilisieren von organischem Material und das mit deren Hilfe gegen oxidativen, thermischen und/oder aktinischen Abbau stabilisierte organische Material.

Organische Materialien, wie z.B. Kunststoffe und Harze unterliegen thermischem, oxidativem und lichtinduziertem Abbau. In der Technik ist eine grosse Anzahl von Stabilisatoren für eine Vielzahl von Substraten bekannt. Die Wirksamkeit eines Stabilisators hängt unter anderem von der Art des Substrats, in welchem er eingesetzt wird, und von jeweiligen Abbaumechanismus ab. Daher ist es im allgemeinen schwierig, für eine konkrete Anwendung den wirksamsten und ökonomischsten Stabilisator anzugeben.

So kann beispielsweise die Wirksamkeit eines Stabilisators, der die Flüchtigkeit einer Verbindung reduziert, darauf zurückgeführt werden, dass er einen Bindungsbruch der Substratmoleküle verhindert. Um eine geringe Versprödung oder die Erhaltung der Elastizität eines Polymeren oder eines Elastomeren zu gewährleisten, kann von einem Stabilisator verlangt werden, dass er übermässige Vernetzungsreaktionen und/oder Kettenabbruch verhindert. Um die Vergilbung zu unterbinden, müssen Reaktionen, die zu neuen Chromophoren führen, verhindert werden. Weiterhin müssen Probleme der Verarbeitungsstabilität und der Substratverträglichkeit beachtet werden.

Verschiedene organische Hydroxylamine sind bekannt une einige sind im Handel erhältlich. Eine Anzahl von Patenten offenbaren substituierte Hydroxylamine als Antioxidantien für verschiedene Substrate, einschliesslich Polyolefine, Polyester und Polyurethane. Als repräsentative Patente seien die US-Patente 3 432 578, 3 644 278, 3 778 464, 3 408 422, 3 926 909, 4 316 996 und 4 386 224 genannt, welche im wesentlichen N,N-Dialkyl-, N,N-Diaryl- und N,N-Diaralkylhydroxylamine und deren Wirksamkeit als Farbverbesserer beschreiben.

Verschiedene Hydroxylamine, die durch Carbamoylgruppen substituiert sind, werden in US-A-3 869 278 und in einem Artikel von Zinner et al. in Pharmazie 20, 291 (1965) beschrieben.

Es wurde nun gefunden, dass die Aminoxypropionamide dieser Erfindung eine Vielzahl der gewünschten Eigenschaften besitzen, wodurch diese Verbindungen zu besonders effektiven Stabilisatoren werden. So schützen sie eine Reihe von Substraten, wie z.B. Polyolefine, Elastomere und Schmiermittel gegen oxidativen und thermischen Abbau. Ferner sind sie sehr wirkungsvolle Farbverbesserer und Verarbeitungsstabilisatoren für Polyolefinzusammensetzungen, die Metallsalze von Fettsäuren und auch phenolische Antioxidantien enthalten können. Hauptsächlich können die erfindungsgemässen Aminoxypropionamide dazu dienen, Verfärbungen zu vermindern, die auf die Gegenwart von phenolischen Antioxidantien und/oder auf die Verarbeitungsbedingungen zurückzuführen sind. Ferner schützen die erfindungsgemässen Verbindungen das organische Polymer direkt vor den Auswirkungen der Verarbeitungsbedingungen. Sie schützen auch solche Polyolefinzusammensetzungen, die gehinderte Amin-Lichtstabilisatoren oder Kombinationen von phenolischen Antioxidantien und Phosphiten enthalten, vor der Verfärbung. Ferner werden Verfärbungen, die auf die Einwirkung von Zerfallsprodukten natürlicher Gase zurückzuführen sind, merklich vermindert.

Die vorliegende Erfindung betrifft Verbindungen der Formel I,

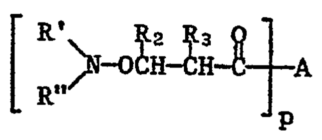

(I)

worin p eine ganze Zahl von 1 bis 4 ist, R′ und R″ unabhängig voneinander Wasserstoff, $C_1$-$C_{36}$-Alkyl, $C_5$-$C_{12}$-Cycloalkyl, $C_7$-$C_9$-Aralkyl oder durch $C_1$-$C_{36}$-Alkyl substituiertes $C_7$-$C_9$-Aralkyl bedeuten, $R_2$ und $R_3$ unabhängig voneinander Wasserstoff oder $C_1$-$C_{12}$-Alkyl sind und
wenn p 1 bedeutet, A eine Gruppe $-NHR_4$ ist, worin $R_4$ Wasserstoff, Amino, $C_1$-$C_{18}$-Alkyl oder

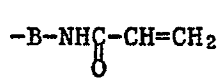

ist und B eine direkte Bindung oder $C_1$-$C_{10}$-Alkylen bedeutet,
wenn p 2 ist, A für einen 5- bis 7-gliedrigen heterocyclischen Rest, der 2 Stickstoffatome mit je einer freien Valenz besitzt, oder für eine Gruppe

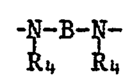

steht, worin B und $R_4$ die oben angegebenen Bedeutungen haben,

wenn p 3 ist, A eine Gruppe der Formel

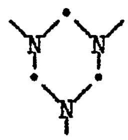 oder $R_5-\underset{|}{N}-(CH_2)_n-\underset{|}{N}-(CH_2)_n-\underset{|}{N}-R_5$

bedeutet, worin n eine ganze Zahl von 2 bis 6 ist und $R_5$ Wasserstoff oder $C_1$-$C_4$-Alkyl ist, und wenn p 4 bedeutet, A eine Gruppe der Formel

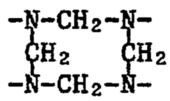 oder $R_5-\underset{|}{N}-(CH_2)_n-\underset{|}{N}-(CH_2)_n-\underset{|}{N}-(CH_2)_n-\underset{|}{N}-R_5$

ist, worin $R_5$ und n die oben angegebenen Bedeutungen haben, unter Ausschluß der Verbindung β-Aminoxypropionamid.

R' und R" bedeuten als $C_1$-$C_{36}$-Alkyl zum Beispiel Methyl, Ethyl, n-Propyl, n-Butyl, tert-Butyl, n-Pentyl, n-Octyl, 2-Ethylhexyl, Decyl, Dodecyl oder Octadecyl. $C_1$-$C_{18}$-Alkyl, welches geradkettig oder verzweigt sein kann, ist bevorzugt. Besonders bevorzugt ist $C_1$-$C_6$-Alkyl, insbesondere Methyl.

R' und R" sind als $C_5$-$C_{12}$-Cycloalkyl beispielsweise Cyclopentyl, Cyclohexyl oder Cyclooctyl. Bevorzugt ist Cycloalkyl mit 5 bis 7 Kohlenstoffatomen, insbesondere Cyclopentyl und Cyclohexyl.

R' und R" können als unsubstituiertes oder durch $C_1$-$C_{36}$-Alkyl substituiertes $C_7$-$C_9$-Aralkyl insbesondere Phenylalkyl mit 7 bis 9 Kohlenstoffatomen bedeuten, wobei der Phenylring gegebenenfalls durch $C_1$-$C_{36}$-Alkyl, bevorzugt Methyl, substituiert sein kann. Beispiele sind Benzyl, α-Methylbenzyl und α,α-Dimethylbenzyl. Benzyl ist besonders bevorzugt.

$R_2$ und $R_3$ bedeuten als $C_1$-$C_{12}$-Alkyl zum Beispiel Methyl, Ethyl, n-Propyl, n-Butyl, tert-Butyl, n-Pentyl, n-Octyl, 2-Ethylhexyl, Decyl oder Dodecyl. $C_1$-$C_4$-Alkyl ist bevorzugt.

$R_4$ besitzt als $C_1$-$C_{18}$-Alkyl beispielsweise die oben für R' und R" als Alkyl angegebenen Bedeutungen.

B bedeutet als $C_1$-$C_{10}$-Alkylen zum Beispiel Methylen, Ethylen, Trimethylen, Tetramethylen, Pentamethylen, Hexamethylen oder Decamethylen. $C_1$-$C_6$-Alkylen, insbesondere geradkettiges, ist bevorzugt.

A bedeutet als 5- bis 7-gliedriger heterocyclischer Rest, der 2 Stickstoffatome mit je einer freien Valenz besitzt, zum Beispiel

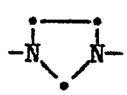 , 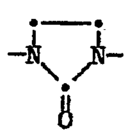 , 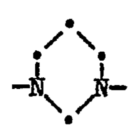 , 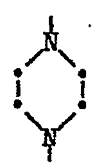 , 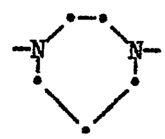 oder

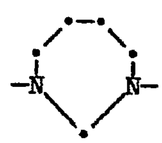 .

$R_5$ is als $C_1$-$C_4$-Alkyl zum Beispiel Methyl, Ethyl, Propyl oder Butyl.

p bedeutet bevorzugt 1 oder 2, insbesondere 1.

$R_2$ und $R_3$ bedeuten bevorzugt Wasserstoff oder $C_1$-$C_4$-Alkyl, insbesondere Wasserstoff.

Wenn p 2 bedeutet, ist A bevorzugt eine Gruppe

$$-\underset{R_4}{\underset{|}{N}}-B-\underset{R_4}{\underset{|}{N}},$$

worin $R_4$ bevorzugt $C_1$-$C_4$-Alkyl, insbesondere Methyl, und B geradkettiges $C_1$-$C_6$-Alkylen darstellen.

Wenn p 3 bedeutet, ist A bevorzugt eine Gruppe

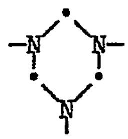.

Bevorzugt sind Verbindungen der Formel I, worin R′ und R″ unabhängig voneinander Wasserstoff, $C_1$-$C_{18}$-Alkyl, Cyclopentyl, Cyclohexyl, Benzyl, α-Methylbenzyl oder α,α-Dimethylbenzyl sind und $R_2$ und $R_3$ unabhängig voneinander Wasserstoff oder $C_1$-$C_4$-Alkyl bedeuten.

R′ und R″ sind bevorzugt Benzyl und $R_2$ und $R_3$ bevorzugt Wasserstoff.

Wenn p 1 ist, ist A bevorzugt eine Gruppe -$NHR_4$, worin $R_4$ $C_1$-$C_{18}$-Alkyl ist.

Bevorzugt sind Verbindungen der Formel I, worin R′ und R″ unabhängig voneinander Benzyl oder $C_1$-$C_6$-Alkyl sind, $R_2$ und $R_3$ Wasserstoff bedeuten und wenn p 1 ist, A eine Gruppe -$NHR_4$ darstellt, worin $R_4$ $C_1$-$C_{18}$-Alkyl ist, und wenn p 2 ist, A eine Gruppe

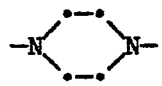 oder 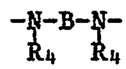

worin B $C_1$-$C_{10}$-Alkylen ist und $R_4$ die oben angegebene Definition besitzt.

Beispiele für bevorzugte Verbindungen der Formel I sind:
N-tert-Butyl-[3-(N′,N′-dibenzylaminoxy)propionamid],
N-tert-Octyl-[3-(N′,N′-dibenzylaminoxy)propionamid],
N,N′-Dimethyl-N,N′-hexamethylen-bis[3-N″,N″-dibenzylaminoxy)propionamid],
1,4-Piperazinyl-bis[3-(N,N-dibenzylaminoxy)propionamid] und
N-tert-Octyl-[3-(N′,N′-diethylaminoxy)propionamid].

Die Verbindungen der Formel I können in Analogie zu bekannten Verfahren hergestellt werden. Wenn p 1 ist, kann die Herstellung zum Beispiel durch Umsetzung einer Verbindung der Formel II,

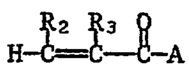 (II)

worin $R_2$, $R_3$ und A die oben für p=1 angegebenen Bedeutungen besitzen, mit einem Hydroxylamin der Formel III,

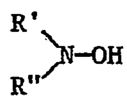 (III)

worin R′ und R″ wie oben definiert sind, gegebenenfalls in einem organischen Lösungsmittel erfolgen.

Typische Beispiele für Verbindungen der Formel II sind Acrylamid, Methacrylamid, N-tert-Butylacrylamid und N-tert-Octylacrylamid.

Als Lösungsmittel seien genannt: Aromatische Kohlenwasserstoffe, wie beispielsweise Benzol, Toluol, Xylol und dergleichen, oder heterocyclische Ether, wie beispielsweise Tetrahydrofuran. Die Reaktionstemperatur liegt zweckmässigerweise bei 65° bis 135°C. Bevorzugt erfolgt die oben beschriebene Umsetzung in Gegenwart eines Katalysators, wie beispielsweise Alkalihydroxid oder Alkalialkoxid.

Wenn p 2, 3 oder 4 ist, erfolgt die Herstellung der Verbindungen der Formel I zum Beispiel durch Umsetzung einer Verbindung der Formel IV

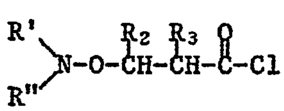 (IV)

mit einer Verbindung der Formel V,

$$A(-H)_p \qquad (V)$$

wobei R′, R″, $R_2$, $R_3$, A und p die oben angegebenen Bedeutungen besitzen.

Typische Beispiele für Verbindungen der Formel V sind für p=2 Pyrazolidin, Imidazolidin, Ethylendiamin, Hexamethylendiamin, N,N′-Dimethyl-hexamethylendiamin, Piperazin und Methylhydrazin, für p=3 Diethylentriamin und s-Hexahydrotriazin und für p=4 Triethylentetramin.

Die Umsetzung findet vorzugsweise in einem organischen Lösungsmittel, wie z.B. Methylenchlorid, und bei Raumtemperatur statt. Die Reagentien können in stöchiometrischem Verhältnis oder mit einem Ueberschuss an einem der beiden Reagentien eingesetzt werden.

Die Verbindungen der Formel IV können in Analogie zu bekannten Verfahren, beispielsweise durch Umsetzung einer Verbindung der Formel VI,

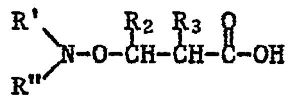

(VI)

worin R′, R″, $R_2$ und $R_3$ die oben angegebenen Bedeutungen haben, mit einem Chlorid, wie beispielsweise Oxalylchlorid oder Thionylchlorid, hergestellt werden. Die Umsetzung findet zweckmässigerweise in einem organischen Lösungsmittel, wie z.B. Methylenchlorid, statt. Die Reaktionstemperatur liegt vorzugsweise bei 0 bis 5°C.

Die Propionsäuren der Formel VI können zweckmässigerweise durch Verseifung der Aklylester hergestellt werden, welche z.B. durch Umsetzung von disubstituierten Hydroxylaminen mit geeigneten Acrylaten erhältlich sind.

Die Ausgangsprodukte für die oben beschriebenen Herstellungsverfahren sind bekannt, teilweise im Handel erhältlich oder können nach bekannten Verfahren hergestellt werden.

Die Verbindungen der Formel I einschließlich der Verbindung β-Aminoxypropionamid eignen sich als Stabilisatoren für eine Reihe von organischen Materialien, wie beispielsweise

1. Polymere von Mono- und Diolefinen, beispielsweise Polyethylen (das gegebenenfalls vernetzt sein kann), Polypropylen, Polyisobutylen, Polybuten-1, Polymethylpenten-1, Polyisopren oder Polybutadien sowie Polymerisate von Cycloolefinen wie z.B. von Cyclopenten oder Norbornen.

2. Mischungen der unter 1) genannten Polymeren, z.B. Mischungen von Polypropylen mit Polyisobutylen.

3. Copolymere von Mono- und Diolefinen untereinander oder mit anderen Vinylmonomeren, wie z.B. Ethylen-Propylen-Copolymere, Propylen-Buten-l-Copolymere, Propylen-Isobutylen-Copolymere, Ethylen-Buten-l-Copolymere, Propylen-Butadien-Copolymere, Isobutylen-Isopren-Copolymere, Ethylen-Alkylacrylat-Copolymere, Ethylen-Alkylmethacrylat-Copolymere, Ethylen-Vinylacetat-Copolymere oder Ethylen-Acrylsäure-Copolymere und deren Salze (Ionomere), sowie Terpolymere von Ethylen mit Propylen und einem Dien, wie Hexadien, Dicyclopentadien oder Ethylidennorbornen.

4. Polystyrol, Poly-(p-methylstyrol).

5. Copolymere von Styrol oder α-Methylstyrol mit Dienen oder Acrylderivaten, wie z.B. Styrol-Butadien, Styrol-Acrylnitril, Styrol-Alkylmethacrylat, Styrol-Maleinanhydrid, Styrol-Acrylnitril- Methylacrylat; Mischungen von hoher Schlagzähigkeit aus Styrol-Copolymeren und einem anderen Polymer, wie z.B. einem Polyacrylat, einem Dien-Polymeren oder einem Ethylen-Propylen-Dien-Terpolymeren; sowie Block-Copolymere des Styrols, wie z.B. Styrol-Butadien-Styrol, Styrol-Isopren-Styrol, Styrol-Ethylen/Butylen-Styrol oder Styrol-Ethylen/Propylen-Styrol.

6. Pfropfcopolymere von Styrol, wie z.B. Styrol auf Polybutadien, Styrol und Acrylnitril auf Polybutadien, Styrol und Maleinsäureanhydrid auf Polybutadien, Styrol und Alkylacrylate bzw. Alkylmethacrylate auf Polybutadien, Styrol und Acrylnitril auf Ethylen-Propylen-Dien-Terpolymeren, Styrol und Acrylnitril auf Polyalkylacrylaten oder Polyalkylmethacrylaten, Styrol und Acrylnitril auf Acrylat-Butadien-Copolymeren, sowie deren Mischungen mit den unter 5) genannten Copolymeren, wie sie z.B. als sogenannte ABS-, MBS-, ASA- oder AES-Polymere bekannt sind.

7. Halogenhaltige Polymere, wie z.B. Polychloropren, Chlorkautschuk, chloriertes oder chlorsulfoniertes Polyethylen, Epichlorhydrinhomo- und -copolymere, insbesondere Polymere aus halogenhaltigen Vinylverbindungen, wie z.B. Polyvinylchlorid, Polyvinylidenchlorid, Polyvinylfluorid, Polyvinylidenfluorid; sowie deren Copolymere, wie Vinylchlorid-Vinylidenchlorid, Vinylchlorid-Vinylacetat oder Vinylidenchlorid-Vinylacetat.

8. Polymere, die sich von α,β-ungesättigten Säuren und deren Derivaten ableiten, wie Polyacrylate und Polymethacrylate, Polyacrylamide und Polyacrylnitrile.

9. Copolymere der unter 8) genannten Monomeren untereinander oder mit anderen ungesättigten Monomeren, wie z.B. Acrylnitril-Butadien-Copolymere, Acrylnitril-Alkylacrylat-Copolymere, Acrylnitril-Alkoxyalkylacrylat-Copolymere, Acrylnitril-Vinylhalogenid-Copolymere oder Acrylnitril-Alkylmethacrylat-Butadien-Terpolymere.

10. Polymere, die sich von ungesättigten Alkoholen und Aminen bzw. deren Acylderivaten oder Acetalen ableiten, wie Polyvinylalkohol, Polyvinylacetat, -stearat, -benzoat, -maleat, Polyvinylbutyral, Polyallylphthalat, Polyallylmelamin.

11. Homo- und Copolymere von cyclischen Ethern, wie Polyalkylenglykole, Polyethylenoxyd, Polypropylenoxyd oder deren Copolymere mit Bisglycidylethern.

12. Polyacetale, wie Polyoxymethylen, sowie solche Polyoxymethylene, die Comonomere, wie z.B. Ethylenoxid, enthalten.

13. Polyphenyloxide und -sulfide und deren Mischungen mit Styrol-polymeren.

14. Polyurethane, die sich von Polyethern, Polyestern und Polybutadienen mit endständigen Hydroxylgruppen einerseits und aliphatischen oder aromatischen Polyisocyanaten andererseits ableiten, sowie deren Vorprodukte.

15. Polyamide und Copolyamide, die sich von Diaminen und Dicarbonsäuren und/oder von Aminocarbonsäuren oder den entsprechenden Lactamen ableiten, wie Polyamid 4, Polyamid 6, Polyamid 6/6, Polyamid 6/10, Polyamid 11, Polyamid 12, Poly-2,4,4-trimethylhexamethylenterephthalamid, Poly-m-phenylenisophthalamid, sowie deren Block-Copolymere mit Polyethern, wie z.B. mit Polyethylenglykol, Polypropylenglykol oder Polytetramethylenglykol.

16. Polyharnstoffe, Polyimide, Polyamid-imide und Polybenzimidazole.

17. Polyester, die sich von Dicarbonsäuren und Dialkoholen und/oder von Hydroxycarbonsäuren oder den entsprechenden Lactonen ableiten, wie Polyethylenterephthalat, Polybutylenterephthalat, Poly-1,4-dimethylolcyclohexanterephthalat, Polyhydroxybenzoate, sowie Block-Polyether-ester, die sich von Polyethern mit Hydroxylendgruppen ableiten.

18. Polycarbonate und Polyestercarbonate.

19. Polysulfone, Polyethersulfone und Polyetherketone.

20. Vernetzte Polymere, die sich von Aldehyden einerseits und Phenolen, Harnstoff oder Melamin andererseits ableiten, wie Phenol-Formaldehyd-, Harnstoff-Formaldehyd- und Melamin-Formaldehydharze.

21. Trocknende und nicht-trocknende Alkydharze.

22. Ungesättigte Polyesterharze, die sich von Copolyestern gesättigter und ungesättigter Dicarbonsäuren mit mehrwertigen Alkoholen, sowie Vinylverbindungen als Vernetzungsmittel ableiten, wie auch deren halogenhaltige, schwerbrennbare Modifikationen.

23. Vernetzbare Acrylharze, die sich von substituierten Acrylsäureestern ableiten, wie z.B. von Epoxyacrylaten, Urethan-acrylaten oder Polyester-acrylaten.

24. Alkydharze, Polyesteharze und Acrylatharze, die mit Melaminharzen, Harnstoffharzen, Polyisocyanaten oder Epoxidharzen vernetzt sind.

25. Vernetzte Epoxidharze, die sich von Polyepoxiden ableiten, z.B. von Bis-glycidylethern oder von cycloaliphatischen Diepoxiden.

26. Natürliche Polymere, wie Cellulose, Naturkautschuk, Gelatine, sowie deren polymerhomolog chemisch abgewandelte Derivate, wie Celluloseacetate, -propionate und -butyrate, bzw. die Celluloseether, wie Methylcellulose.

27. Mischungen (Polyblends) der vorgenannten Polymeren, wie z.B. PP/EPDM, Polyamid 6/EPDM oder ABS, PVC/EVA, PVC/ABS, PVC/MBS, PC/ABS, PBTP/ABS.

28. Natürliche und synthetische organische Stoffe, die reine monomere Verbindungen oder Mischungen von solchen darstellen, beispielsweise Mineralöle, tierische oder pflanzliche Fette, Oele und Wachse, oder Öle, Wachse und Fette auf Basis synthetischer Ester (z.B. Phthalate, Adipate, Phosphate oder Trimellithate), sowie Abmischungen synthetischer Ester mit Mineralölen in beliebigen Gewichtsverhältnissen, wie sie z.B. als Spinnpräparationen Anwendung finden, sowie deren wässrige Emulsionen.

29. Wässrige Emulsionen natürlicher oder synthetischer Kautschuke, wie z.B. Naturkautschuk-Latex oder Latices von carboxylierten Styrol-Butadien-Copolymeren.

Die erfindungsgemässen Verbindungen sind besonders wirksame Stabilisatoren für Kunststoffe, Polymere und Harze.

Bevorzugt werden die Verbindungen der Formel I in synthetischen Polymeren, insbesondere Polyolefin-Homopolymeren oder -Copolymeren, eingesetzt.

Besonders bevorzugt werden die Verbindungen der Formel I in Polyolefinen, wie beispielsweise Polyethylen und Polypropylen, Polystyrol, einschliesslich schlagfestem Polystyrol, Acrylnitril-Butadien-Styrol-Harz (ABS), Styrol-Butadien-Kautschuk (SBR), Isopren als auch natürlich Kautschuk, Polyester, einschliesslich Polyethylenterephthalat und Polybutadienterephthalat sowie deren Copolymeren, und in Schmierölen, die beispielsweise aus Mineralölen hergestellt werden, eingesetzt.

Im allgemeinen können die erfindungsgemässen Verbindungen in Konzentrationen von ~0,01 bis ~5 Gew.-%, bezogen auf die stabilisierte Zusammensetzung, eingesetzt werden; allerdings kann je nach Verwendung und Substrat die Stabilisatormenge variieren. Bevorzugt verwendet man ~0,5 bis 2 Gew.-%, insbesondere 0,1 bis 1 Gew.-%.

Die Einarbeitung der erfindungsgemässen Stabilisatoren in das organische Material kann nach bekannten Methoden während jeder Verarbeitungsstufe vor der Formgebung erfolgen. Der Stabilisator kann beispielsweise in Pulverform dem organischen Material zugemischt werden oder eine Emulsion bzw. Suspension des Stabilisators kann mit einer Lösung, Suspension oder Emulsion des organischen Materials vermischt werden.

Die stabilisierten Zusammensetzungen dieser Erfindung können gegebenenfalls auch herkömmliche Additive enthalten.

Beispiele für herkömmliche Additive sind:

1. Antioxidantien

1.1. Alkylierte Monophenole, z.B. 2,6-Di-tert.butyl-4-methylphenol, 2-Tert.butyl-4,6-dimethylphenol, 2,6-Di-tert.butyl-4-ethylphenol, 2,6-Di-tert.butyl-4-n-butylphenol, 2,6-Di-tert.butyl-4-i-butylphenol, 2,6-Di-cyclopentyl-4-methylphenol, 2-(α-Methylcyclohexyl)-4,6-dimethylphenol, 2,6-Di-octadecyl-4-methylphenol, 2,4,6-Tricyclohexylphenol, 2,6-Di-tert.butyl-4-methoxymethylphenol.

1.2. Alkylierte Hydrochinone, z.B. 2,6-Di-tert.butyl-4-methoxyphenol, 2,5-Di-tert.butyl-hydrochinon, 2,5-Di-tert.amyl-hydrochinon, 2,6-Diphenyl-4-octadecyloxyphenol.

1.3. Hydroxylierte Thiodiphenylether, z.B. 2,2'-Thio-bis-(6-tert.butyl-4-methylphenol), 2,2'-Thio-bis-(4-octylphenol), 4,4'-Thio-bis-(6-tert.butyl-3-methylphenol), 4,4'-Thio-bis-(6-tert.butyl-2-methylphenol).

1.4. Alkyliden-Bisphenole, z.B. 2,2'-Methylen-bis-(6-tert.butyl-4-methylphenol), 2,2'-Methylen-bis-(6-tert.butyl-4-ethylphenol), 2,2'-Methylen-bis-[4-methyl-6-(α-methylcyclohexyl)-phenol], 2,2'-Methylen-bis-(4-methyl-6-cyclohexylphenol), 2,2'-Methylen-bis-(6-nonyl-4-methylphenol), 2,2'-Methylen-bis-(4,6-di-tert.butylphenol), 2,2'-Ethyliden-bis-(4,6-di-tert.butylphenol), 2,2'-Ethyliden-bis-(6-tert.butyl-4-isobutylphenol), 2,2'-Methylen-bis-[6-(α-methylbenzyl)-4-nonylphenol], 2,2'-Methylen-bis-[6-(α,α-dimethylbenzyl)-4-nonylphenol], 4,4'-Methylen-bis-(2,6-di-tert.butylphenol), 4,4'-Methylen-bis-(6-tert.butyl-2-methylphenol), 1,1-Bis-(5-tert.butyl-4-hydroxy-2-methylphenyl)-butan, 2,6-Di-(3-tert.butyl-5-methyl-2-hydroxybenzyl)-4-methylphenol, 1,1,3-Tris-(5-tert.butyl-4-hydroxy-2-methylphenyl)-butan, 1,1-Bis-(5-tert.butyl-4-hydroxy-2-methylphenyl)-3-n-dodecylmercaptobutan, Ethylenglycol-bis-[3,3-bis-(3'-tert.butyl-4'-hydroxyphenyl)-butyrat], Di-(3-tert.butyl-4-hydroxy-5-methylphenyl)-dicyclopentadien, Di-[2-(3'-tert.butyl-2'-hydroxy-5'-methyl-benzyl)-6-tert.butyl-4-methyl-phenyl]-terephthalat.

1.5. Benzylverbindungen, z.B. 1,3,5-Tri-(3,5-di-tert.butyl-4-hydroxybenzyl)-2,4,6-trimethylbenzol, Di-(3,5-di-tert.butyl-4-hydroxybenzyl)-sulfid, 3,5-Di-tert.butyl-4-hydroxybenzyl-mercaptoessigsäure-isooctylester, Bis-(4-tert.butyl-3-hydroxy-2,6-dimethylbenzyl)dithiol-terephthalat, 1,3,5-Tris-(3,5-di-tert.butyl-4-hydroxybenzyl)-isocyanurat, 1,3,5-Tris-(4-tert.butyl-3-hydroxy-2,6-dimethylbenzyl)-isocyanurat, 3,5-Di-tert.butyl-4-hydroxybenzyl-phosphonsäure-dioctadecylester, 3,5-Di-tert.butyl-4-hydroxybenzyl-phosphonsäure-monoethylester, Calcium-salz.

1.6. Acylaminophenole, z.B. 4-Hydroxy-laurinsäureanilid, 4-Hydroxystearinsäureanilid, 2,4-Bis-octylmercapto-6-(3,5-di-tert.butyl-4-hydroxyanilino)-s-triazin, N-(3,5-di-tert.butyl-4-hydroxyphenyl)-carbaminsäureoctylester.

1.7. Ester der ß-(3,5-Di-tert.butyl-4-hydroxyphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-hydroxyethyl-isocyanurat, Di-hydroxyethyl-oxalsäurediamid.

1.8. Ester der ß-(5-tert.Butyl-4-hydroxy-3-methylphenyl)-propionsäure mit ein- oder mehrwertigen Alkoholen, wie z.B. mit Methanol, Octadecanol, 1,6-Hexandiol, Neopentylglycol, Thiodiethylenglycol, Diethylenglycol, Triethylenglycol, Pentaerythrit, Tris-hydroxyethyl-isocyanurat, Di-hydroxyethyl-oxalsäurediamid.

1.9. Amide der ß-(3,5-Di-tert.butyl-4-hydroxyphenyl)-propionsäure, wie z.B. N,N'-Di-(3,5-di-tert.butyl-4-hydroxyphenylpropionyl)-hexamethylendiamin, N,N'-Di-(3,5-di-tert.butyl-4-hydroxyphenylpropionyl)-trimethylendiamin, N,N'-Di-(3,5-di-tert.butyl-4-hydroxyphenylpropionyl)-hydrazin.

2. UV-Absorber und Lichtschutzmittel

2.1. 2-(2'-Hydroxyphenyl)-benztriazole, wie z.B. das 5'-Methyl-, 3',5'-Di-tert.butyl-, 5'-tert.Butyl-, 5'-(1,1,3,3-Tetramethylbutyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydroxybenzophenone, wie z.B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z.B. 4-tert.Butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäurehexadecylester.

2.4. Acrylate, wie z.B. α-Cyan-ß,ß-diphenylacrylsäure-ethylester bzw. -isooctylester, α-Carbomethoxy-zimtsäuremethylester, α-Cyano-ß-methyl-p-methoxy-zimtsäuremethylester bzw. -butylester, α-Carbomethoxy-p-methoxy-zimtsäure-methylester, N-(ß-Carbomethoxy-ß-cyanovinyl)-2-methyl-indolin.

2.5. Nickelverbindungen, wie z.B. Nickelkomplexe des 2,2'-Thio-bis-[4-(1,1,3,3-tetramethylbutyl)-phenols], wie der 1:1- oder der 1:2-Komplex, gegebenenfalls mit zusätzlichen Liganden, wie n-Butylamin, Triethanolamin oder N-Cyclohexyl-diethanolamin, Nickeldibutyl-dithiocarbamat, Nickelsalze von 4-Hydroxy-3,5-di-tert.butylbenzylphosphonsäure-monoalkylester, wie vom Methyl- oder Ethylester, Nickelkomplexe von Ketoximen, wie von 2-Hydroxy-4-methyl-phenylundecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z.B. Bis-(2,2,6,6-tetramethylpiperidyl)-sebacat, Bis-(1,2,2,6,6-pentamethylpiperidyl)-sebacat, n-Butyl-3,5-di-tert.butyl-4-hydroxybenzyl-malonsäure-bis(1,2,2,6,6-pentamethylpiperidyl)-ester, Kondensationsprodukt aus 1-Hydroxyethyl-2,2,6,6-tetramethyl-4-hydroxypiperidin und Bernsteinsäure, Kondensationsprodukt aus N,N'-(2,2,6,6-Tetramethyl-4-piperidyl)-hexamethylendiamin und 4-tert.Octylamino-2,6-dichlor-1,3,5-s-triazin, Tris-(2,2,6,6-tetramethyl-4-piperidyl)-nitrilotriacetat, Tetrakis-(2,2,6,6-tetramethyl-4-piperidyl)-1,2,3,4-butantetracarbonsäure, 1,1'-(1,2-Ethandiyl)-bis-(3,3,5,5-tetramethyl-piperazinon).

2.7. Oxalsäurediamide, wie z.B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-di-methylaminopropyl)-oxalamid, 2-Ethoxy-5-tert.butyl-2'-ethyloxanilid und dessen Gemisch mit 2-Ethoxy-2'-ethyl-5,4'-di-tert.butyl-oxanilid, Gemische von o-und p-Methoxy- sowie von o- und p-Ethoxy-di-substituierten Oxaniliden.

3. Metalldesaktivatoren, wie z.B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z.B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit, 3,9-Bis-(2,4-di-tert.butylphenoxy-2,4,8,10-tetraoxa-3,9-diphosphaspiro[5.5]undecan.

5. Peroxidzerstörende Verbindungen, wie z.B. Ester der ß-Thio-dipropionsäure, beispielsweise der Lauryl-, Stearyl-, Myristyl- oder Tridecylester, Mercaptobenzimidazol, das Zinksalz des 2-Mercaptobenzimidazols, Zink-dibutyl-dithiocarbamat, Dioctadecyldisulfid, Pentaerythrit-tetrakis-(ß-dodecylmercapto)-propionat.

6. Polyamidstabilisatoren, wie z.B. Kupfersalze in Kombination mit Jodiden und/oder Phosphorverbindungen und Salze des zweiwertigen Mangans.

7. Basische Co-Stabilisatoren, wie z.B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z.B. 4-tert.Butylbenzoesäure, Adipinsäure, Diphenylessigsäure.

9. Füllstoffe und Verstärkungsmittel, wie z.B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z.B. Weichmacher, Gleitmittel, Emulgatoren, Pigmente, Optische Aufheller, Flammschutzmittel, Antistatika, Treibmittel.

Bevorzugt enthalten die erfindungsgemässen Zusammensetzungen als weitere Zusätze ein Mettalsalz einer höheren Fettsäure und/oder ein phenolisches Antioxidans.

Die Verbindungen der Formel I eignen sich sowohl allein als auch in Kombination mit anderen Additiven als Stabilisatoren für eine Reihe von Substraten, insbesondere Polyolefinen, die gegebenenfalls Alkalimetall-, Erdalkalimettall- oder Aluminiumsalze von höheren Fettsäuren (Beispiele sind die oben unter Punkt 7 aufgeführten Additive) und/oder gehinderte phenolische Antioxidantien enthalten können. Die erfindungsgemässen Aminoxypropionamide vermindern sehr wirksam Verfärbungen, die auf die Anwesenheit von Phenolen zurückzuführen sind. Solche phenolischen Antioxidantien sind beispielsweise: n-Octadecyl-3,5-di-tert-butyl-4-hydroxyhydrocinnamat, Neopentantetrayl-tetrakis(3,5-di-tert-butyl-4-hydroxy-hydrocinnamat), Di-n-octadecyl-3,5-di-tert-butyl-4-hydroxybenzyl-phosphonat, 1,3,5-Tris(3,5-di-tert-butyl-4-hydroxybenzyl)isocyanurat, Thiodiethylen-bis(3,5-di-tert-butyl-4-hydroxyhydrocinnamat), 1,3,5-Trimethyl-2,4,6-tris(3,5-di-tert-butyl-4-hydroxybenzyl)benzol, 3,6-Dioxaoctamethylen-bis(3-methyl-5-tert-butyl-4-hydroxyhydrocinnamat), 2,6-Di-tert-butyl-p-cresol, 2,2'-Ethyliden-bis(4,6-di-tert-butylphenol), 1,3,5-Tris(2,6-dimethyl-4-tert-butyl-3-hydroxybenzyl)isocyanurat, 1,1,3-Tris(2-methyl-4-hydroxy-5-tert-butylphenyl)butan, 1,3,5-Tris[2-(3,5-di-tert-butyl-4-hydroxyhydrocinnamoyloxy)ethyl]isocyanurat, 3,5-Bis(3,5-di-tert-butyl-4-hydroxybenzyl)mesitol, Hexamethylen-bis(3,5-di-tert-butyl-4-hydroxyhydrocinnamat), 1-(3,5-Di-tert-butyl-4-hydroxyanilino)-3,5-bis(octylthio)-s-triazin, N,N'-Hexamethylen-bis(3,5-di-tert-butyl-4-hydroxyhydrocinnamamid), Kalzium-bis(ethyl-3,5-di-tert-butyl-4-hydroxybenzylphosphonat), Ethylen-bis[3,3-bis(3-tert-butyl-4-hydroxyphenyl)butyrat], Octyl-3,5-di-tert-butyl-4-hydroxybenzylmercaptoacetat, Bis(3,5-di-tert-butyl-4-hydroxyhydrocinnamoyl)hydrazid und N,N'-Bis[2-(3,5-di-tert-butyl-4-hydroxyhydrocinnamoyloxy)ethyl]oxamid, insbesondere Neopentantetrayl-tetrakis(3,5-di-tert-butyl-4-hydroxyhydrocinnamat), n-Octadecyl-3,5-di-tert-butyl-4-hydroxyhydrocinnamat), 1,3,5-Trimethyl-2,4,6-tris(3,5-di-tert-butyl-4-hydroxybenzyl)benzol, 1,3,5-Tris(3,5-di-tert-butyl-4-hydroxybenzyl)isocyanurat, 2,6-Di-tert-butyl-p-cresol oder 2,2'-Ethyliden-bis(4,6-di-tert-butylphenol).

Die Verbindungen der Formel I verhindern auch Verfärbungen, die auf die Anwesenheit von gehinderten Amin-Lichtstabilisatoren zurückzuführen sind, wie beispielsweise

Bis(1,2,2,6,6-pentamethyl-4-piperidyl)-2-n-butyl-2-(3,5-di-tert-butyl-4-hydroxybenzyl)malonat, Bis(2,2,6,6-tetramethyl-4-piperidyl)-sebacat, Umsetzungsprodukt von Dimethylsuccinat mit 1-Hydroxyethyl-2,2,6,6-tetramethyl-4-hydroxypiperidin und Umsetzungsprodukt von 2,4-Dichloro-6-octylamino-s-triazin mit N,N'-(2,2,6,6-Tetramethyl-4-piperidyl)hexamethylendiamin.

Die folgenden Beispiele erläutern die Erfindung weiter. Alle Mengenangaben beziehen sich auf das Gewicht, soweit nicht anders angegeben.

Beispiel 1: Herstellung von N-tert-Butyl-[3-N',N'-dibenzylaminoxy)propionamid] (Verbindung 1)

Eine Lösung von 21,33 g Dibenzylhydroxylamin in 100 ml trockenem Tetrahydrofuran wird mit 1,12 g Kalium-tert-butoxid und anschliessend mit 12,70 g N-tert-Butylacrylamid gemischt. Die Lösung wird 24 Stunden unter Rückfluss in einer Stickstoffatmosphäre erhitzt. Das Reaktionsgemisch wird unter vermindertem Druck konzentriert und der Rückstand wird mit einem Wasser/Methylenchlorid-Gemisch aufgenommen. Die organische Schicht wird mit Wasser und einer Kochsalzlösung gewaschen, über $MgSO_4$ getrocknet und bei vermindertem Druck eingeengt. Das Rohprodukt wird chromatographisch gereinigt (Flüssigkeitschromatographie). Das Produkt liegt als Öl vor.

Elementaranalyse:

Berechnet für $C_{21}H_{28}N_2O_2$: C 74,1 %; H 8,3 %; N 8,2 %
Gefunden: C 74,3 %; H 8,5 %; N 8,3 %

Beispiel 2: Herstellung von N-tert-Octyl-[3-(N',N'-dibenzylaminoxy) propionamid] (Verbindung 2)

Die Herstellung erfolgt in Analogie zu Beispiel 1. Es werden 23,31 g Dibenzylhydroxylamin, 1,23 g Kalium-tert-butoxid und 20,0 g N-tert-Octylacrylamid in 100 ml trockenem Tetrahydrofuran eingesetzt. Das Produkt liegt als weisser kristalliner Feststoff mit einem Schmelzpunkt von 96-98°C vor.

Elementaranalyse:

Berechnet für $C_{24}H_{36}N_2O_2$: C 75,0 %; H 9,4 %; N 7,3 %
Gefunden: C 75,6 %; H 9,1 %; N 7,2 %

Beispiel 3: Hestellung von N,N'-Dimethyl-N,N'-hexamethylen-bis-[3-(N",N"-dibenzylaminoxy)propionamid] (Verbindung 3)

Eine Lösung 6,4 g 3-(N,N-Dibenzylaminoxy)propionsäure in 25 ml Methylenchlorid wird mit 1,8 ml Oxalychlorid bei 0-5°C gemischt. Nach 2 Stunden wird bei 0°C eine Lösung von 8,61 g N,N'-Dimethylhexamethylendiamin in 25 ml Methylenchlorid hinzugegeben und das Reaktionsgemisch wird 12 Stunden bei Raumtemperatur gerührt. Der unlösliche Rückstand wird abfiltriert und das Filtrat wird weiterverarbeitet. Das Methylenchlorid wird im Vakuum entfernt und das Produkt wird chromatographisch (Flüssigkeitschromatographie) gereinigt. Man erhält ein dickes Öl.

Beispiel 4: Herstellung von 1,4-Piperazinyl-bis[3-(N,N-dibenzylaminoxy)propionamid] (Verbindung 4)

Die Herstellung erfolgt in Analogie zu Beispiel 3. Es werden 3,78 g der Säure, 1,1 ml Oxalylchlorid und 2,02 g Piperazin eingesetzt. Das Produkt liegt als Öl vor.

Elementaranalyse:

Berechnet für $C_{38}H_{44}N_4O_4$: C 73,5 %; H 7,1 %; N 9,0 %
Gefunden: C 73,2 %; H 7,6 %; N 8,6 %

Beispiel 5: Herstellung von N-tert-Octyl-[3-N',N'-diethylaminoxy)propionamid] (Verbindung 5)

Die Herstellung erfolgt in Analogie zu Beispiel 1. Es werden Diethylhydroxylamin, Kalium-tert-butoxid und N-tert-Octylacrylamid in Tetrahydrofuran eingesetzt.

Elementaranalyse:

Berechnet für $C_{15}H_{32}N_2O_2$: C 66,1 %; H 11,8 %; N 10,3 %
Gefunden: C 66,1 %; H 11,7 %; N 10,3 %

Beispiel 6: Verarbeitungsstabilität von Polypropylen

Die Grundformulierung enthält 100 Teile unstabilisiertes Polypropylen (®Profax 6501, Himont) und 0,1 Teil Kalziumstearat. Die unten angegebenen Stabilisatoren werden in Form einer Lösung dem Polypropylen zugemischt (Lösungsmittel: Methylenchlorid). Das Lösungsmittel wird im Vakuum entfernt. Die stabilisierte Harzformulierung wird bei 100 Umdrehungen pro Minute extrudiert.

Extrusionsbedingungen:

Zylinder 1 232°C
Zylinder 2 246°C
Zylinder 3 260°C

Düse 1 260°C
Düse 2 260°C
Düse 3 260°C

Nach der ersten, dritten und fünften Extrusion werden die Harz-Kügelchen unter Druck bei 193°C zu 3,2 mm dicken Folien verformt.

Der "Yellowness Index" der Proben wird gemäss ASTM D 1925-63 T bestimmt. Niedrige Werte bedeuten eine geringe Verfärbung. Die Ergebnisse sind in den folgenden Tabellen wiedergegeben.

Der Schmelzindex der Proben wird gemäss ASTM 1238-L bestimmt. Höhere Werte bedeuten eine geringere Molmasse und sind ein Hinweis dafür, dass das Polymer abgebaut ist. Die Ergebnisse der verschiedenen Testserien sind in den folgenden Tabellen aufgeführt.

Serie 1:

| Stabilisator | Extrusionstemperatur 260°C "Yellowness Index" nach der | | |
|---|---|---|---|
| | 1. Extrusion | 3. Extrusion | 5. Extrusion |
| ohne | 2,5 | 3,5 | 4,7 |
| 0,1 % Antioxidans A | 7,4 | 13,2 | 15,9 |
| 0,1 % Antioxidans A plus 0,05 % Verbindung 1 | 3,3 | 3,3 | 3,5 |

| Stabilisator | "Schmelzindex" in g/10 min nach der | |
|---|---|---|
| | 1. Extrusion | 5. Extrusion |
| ohne | 6,3 | 14,9 |
| 0,1 % Antioxidans A | 3,4 | 6,9 |
| 0,1 % Antioxidans A plus 0,05 % Verbindung 1 | 3,1 | 4,7 |

Serie 2:

| | Extrusionstemperatur 260°C "Yellowness Index" nach der | | |
|---|---|---|---|
| Stabilisator | 1. Extrusion | 3. Extrusion | 5. Extrusion |
| ohne | 3,6 | 3,9 | 4,6 |
| 0,1 % Antioxidans A | 6,1 | 7,9 | 9,4 |
| 0,1 % Antioxidans A plus 0,05 % Verbindung 2 | 2,0 | 3,5 | 4,0 |

Antioxidans A: Neopentyl-tetrakis[3-(3′,5′-di-tert-butyl-4-hydroxyphenyl)propanoat]

Beispiel 7: Lichstabilität von Polypropylen

Unstabilisiertes Polypropylenpulver (®Hercules Profax 6501) wird mit dem in Tabelle 1 angegebenen Additiv gemischt. Diese Mischung wird bei 182°C 5 Minuten auf einem Mischwalzwerk plastifiziert. Dann löst man die stabilisierte Polypropylenfolie vom Walzwerk ab und lässt sie abkühlen. Die Folie wird in Stücke geschnitten, welche mit einer hydraulischen Presse bei 250°C und 12 bar zu 0,635 mm dicken Filmen verformt werden.

Die Proben werden in einer "fluorescent sunlight/black light chamber" (FS/BL) belichtet. Der Carbonylgehalt der Prüflinge wird mit IR-Spektroskopie bestimmt. Als Mass für die Wirksamkeit des Stabilisators gilt die Zeit bis zum Erreichen einer Carbonylabsorption von 0,5. Die Ergebnisse sind in Tabelle 1 aufgeführt.

Tabelle 1:

| Stabilisator | Stunden bis zum Erreichen einer Carbonylabsorption von 0,5 |
|---|---|
| ohne | 100 |
| 0,2 % Verbindung 1 | 220 |
| 0,2 % Verbindung 2 | 330 |
| 0,2 % Verbindung 4 | 320 |

## Patentansprüche

1. Verbindungen der Formel I,

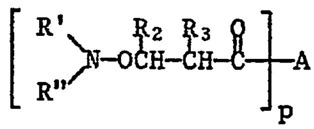

(I)

worin p eine ganze Zahl von 1 bis 4 ist, R′ und R″ unabhängig voneinander Wasserstoff, $C_1$-$C_{36}$-Alkyl, $C_5$-$C_{12}$-Cycloalkyl, $C_7$-$C_9$-Aralkyl oder durch $C_1$-$C_{36}$-Alkyl substituiertes $C_7$-$C_9$-Aralkyl bedeuten, $R_2$ und $R_3$ unabhängig voneinander Wasserstoff oder $C_1$-$C_{12}$-Alkyl sind und
wenn p 1 bedeutet, A eine Gruppe $-NHR_4$ ist, worin $R_4$ Wasserstoff, Amino, $C_1$-$C_{18}$-Alkyl oder

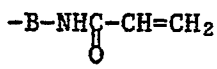

ist und B eine direkte Bindung oder $C_1$-$C_{10}$-Alkylen bedeutet,
wenn p 2 ist, A für einen 5-bis 7-gliedrigen heterocyclischen Rest, der 2 Stickstoffatome mit je einer freien Valenz besitzt, oder für eine Gruppe

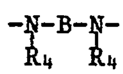

steht, worin B und $R_4$ die oben angegebenen Bedeutungen haben,
wenn p 3 ist, A eine Gruppe der Formel

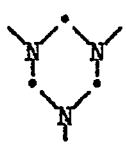

oder

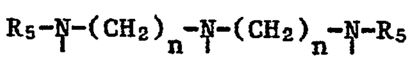

bedeutet, worin n eine ganze Zahl von 2 bis 6 ist und $R_5$ Wasserstoff oder $C_1$-$C_4$-Alkyl ist, und
wenn p 4 bedeutet, A eine Gruppe der Formel

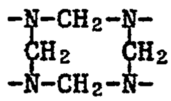

oder

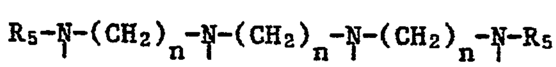

ist, worin $R_5$ und n die oben angegebenen Bedeutungen haben unter Ausschluß der Verbindung β-Aminoxypropionamid.

2. Verbindungen gemäss Anspruch 1, worin R′ und R″ unabhängig voneinander Wasserstoff, $C_1$–$C_{18}$-Alkyl, Cyclopentyl, Cyclohexyl, Benzyl, α-Methylbenzyl oder α,α-Dimethylbenzyl sind und $R_2$ und $R_3$ unabhängig voneinander Wasserstoff oder $C_1$–$C_4$-Alkyl bedeuten.

3. Verbindungen gemäss Anspruch 1, worin R′ und R″ Benzyl sind und $R_2$ und $R_3$ Wasserstoff bedeuten.

4. Verbindungen gemäss Anspruch 1, worin p 1 ist, A eine Gruppe $-NHR_4$ bedeutet und $R_4$ $C_1$–$C_{18}$-Alkyl ist.

5. Verbindungen gemäss Anspruch 1, worin p 2 ist und A oder

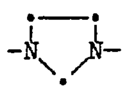 , 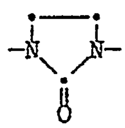 , 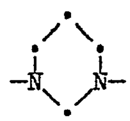 , 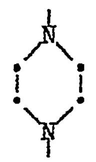 , 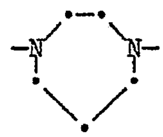 oder

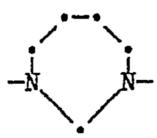

bedeutet.

6. Verbindungen gemäss Anspruch 1, worin p 3 ist und A

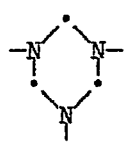

bedeutet.

7. Verbindungen gemäss Anspruch 1, worin R′ und R″ unabhängig voneinander Benzyl oder $C_1$–$C_6$-Alkyl sind, $R_2$ und $R_3$ Wasserstoff bedeuten und wenn p 1 ist, A eine Gruppe $-NHR_4$ darstellt, worin $R_4$ $C_1$–$C_{18}$-Alkyl ist, und wenn p 2 ist, A eine Gruppe

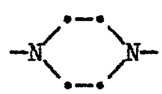 oder 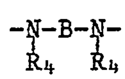

bedeutet, worin B $C_1$–$C_{10}$-Alkylen ist und $R_4$ die oben angegebene Definition besitzt.

8. Die Verbindungen
N-tert-Butyl-[3-(N′,N′-dibenzylaminoxy)propionamid],
N-tert-Octyl-[3-(N′,N′-dibenzylaminoxy)propionamid],
N,N′-Dimethyl-N,N′-hexamethylen-bis[3-(N″,N‴-dibenzylaminoxy)-propionamid],
1,4-Piperazinyl-bis[3-(N,N-dibenzylaminoxy)propionamid] urd
N-tert-Octyl-[3-(N′,N′-diethylaminoxy)propionamid]
gemäss Anspruch 1.

9. Zusammensetzung enthaltend ein gegen oxidativen, thermischen und/oder aktinischen Abbau empfindliches organisches Material und mindestens eine Verbindung der Formel I,

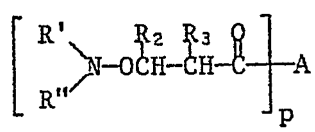 (I)

worin p eine ganze Zahl von 1 bis 4 ist, R′ und R″ unabhängig voneinander Wasserstoff, $C_1$–$C_{36}$-Alkyl, $C_5$–$C_{12}$-Cycloalkyl, $C_7$–$C_9$-Aralkyl oder durch $C_1$–$C_{36}$-Alkyl substituiertes $C_7$–$C_9$-Aralkyl bedeuten, $R_2$ und $R_3$ unabhängig voneinander Wasserstoff oder $C_1$–$C_{12}$-Alkyl sind und wenn p 1 bedeutet, A eine Gruppe $-NHR_4$ ist, worin $R_4$ Wasserstoff, Amino, $C_1$–$C_{18}$-Alkyl oder

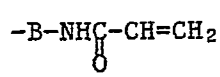

ist und B eine direkte Bindung oder $C_1$–$C_{10}$-Alkylen bedeutet,

wenn p 2 ist, A für einen 5- bis 7gliedrigen heterocyclischen Rest, der 2 Stickstoffatome mit je einer freien Valenz besitzt, oder für eine Gruppe

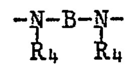

steht, worin B und $R_4$ die oben angegebenen Bedeutungen haben,
wenn p 3 ist, A eine Gruppe der Formel

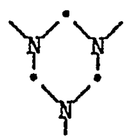 oder 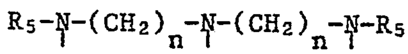

bedeutet, worin n eine ganze Zahl von 2 bis 6 ist und $R_5$ Wasserstoff oder $C_1$–$C_4$-Alkyl ist, und
wenn p 4 bedeutet, A eine Gruppe der Formel

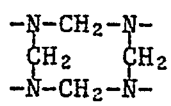 oder 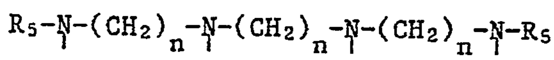

ist, worin $R_5$ und n die oben angegebenen Bedeutungen haben.

10. Zusammensetzung gemäss Anspruch 9, worin das organische Material ein synthetisches Polymer ist.

11. Zusammensetzung gemäss Anspruch 10, worin das synthetische Polymer ein Polyolefin-Homopolymer oder -Copolymer ist.

12. Zusammensetzung gemäss Anspruch 9, dadurch gekennzeichnet, dass die Zusammensetzung mindestens ein herkömmliches Additiv enthält.

13. Zusammensetzung gemäss Anspruch 12, worin das herkömmliche Additiv ein Metallsalz einer höheren Fettsäure ist.

14. Zusammensetzung gemäss Anspruch 12, worin das herkömmliche Additiv ein phenolisches Antioxidans ist.

15. Zusammensetzung gemäss Anspruch 12, worin die herkömmlichen Additive ein Metallsalz einer höheren Fettsäure und ein phenolisches Antioxidans sind.

16. Zusammensetzung gemäss Anspruch 14, worin das phenolische Antioxidans Neopentantetrayl-tetrakis(3,5-di-tert-butyl-4-hydroxyhydrocinnamat), n-Octadecyl-3,5-di-tert-butyl-4-hydroxyhydrocinnamat, 1,3,5-Trimethyl-2,4,6-tris(3,5-di-tert-butyl-4-hydroxybenzyl)benzol, 1,3,5-tris(3,5-di-tert-butyl-4-hydroxybenzyl)isocyanurat, 2,6-Ditert-butyl-p-cresol oder 2,2′-Ethyliden-bis(4,6-di-tert-butylphenol) ist.

17. Verwendung der in Anspruch 9 definierten Verbindungen der Formel I von organischem Material gegen oxidativen, thermischen und/oder aktinischen Abbau.

**Revendications**

1. Composés répondant à la formule I:

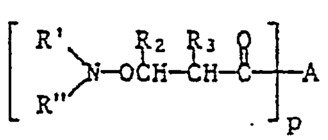 (I)

dans laquelle:
p désigne un nombre entier de 1 à 4,
R′ et R″ représentent chacun, indépendamment l'un de l'autre, l'hydrogène, un alkyle en $C_1$–$C_{36}$, un cycloalkyle en $C_5$–$C_{12}$, un aralkyle en $C_7$–$C_9$ ou un aralkyle en $C_7$–$C_9$ porteur d'un alkyle en $C_1$–$C_{36}$,
$R_2$ et $R_3$ représentent chacun, indépendamment l'un de l'autre, l'hydrogène ou un alkyle en $C_1$–$C_{12}$, et
A représente:
– dans le cas où p est égal à 1, un radical -$NHR_4$ dans lequel $R_4$ représente l'hydrogène, un amino, un alkyle en $C_1$–$C_{18}$ ou un radical

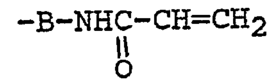

et B représente une liaison directe ou un alkylène en $C_1$–$C_{10}$,
– dans le cas où p est égal à 2, un radical hétérocyclique comportant de 5 à 7 maillons et contenant deux atomes d'azote ayant chacun une liaison libre, ou un radical

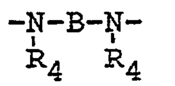

dans lequel B et $R_4$ ont les significations qui leur ont été données ci-dessus,
– dans le cas où p est égal à 3, un radical de formule

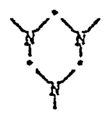

ou un radical de formule:

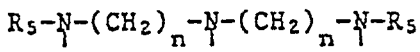

dans lequel n désigne un nombre entier de 2 à 6 et $R_5$ représente l'hydrogène ou un alkyle en $C_1$–$C_4$, et
– dans le cas où p est égal à 4, un radical de formule

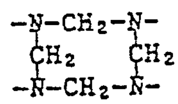

ou un radical de formule:

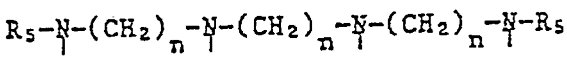

dans lequel $R_5$ et n ont les significations qui leur ont eté données ci-dessus,
sauf l'(amino-oxy)-3 propionamide.

2. Composés selon la revendication 1 dans lesquels R′ et R″ représentent chacun, indépendamment l'un de l'autre, l'hydrogène, un alkyle en $C_1$–$C_{18}$, un cyclopentyle, un cyclohexyle, un benzyle, un α-méthylbenzyle ou un α,α-diméthyl-benzyle, et $R_2$ et $R_3$ représentent chacun, indépendamment l'un de l'autre, l'hydrogène ou un alkyle en $C_1$–$C_4$.

3. Composés selon la revendication 1 dans lesquels R′ et R″ représentent chacun un benzyle, et $R_2$ et $R_3$ chacun l'hydrogène.

4. Composés selon la revendication 1 dans lesquels p est égal à 1, A représente un radical $-NHR_4$ et $R_4$ représente un alkyle en $C_1$–$C_{18}$.

5. Composés selon la revendication 1 dans lesquels p est égal à 2 et A représente l'un des radicaux suivants:

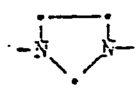 , 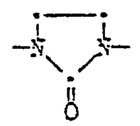 , 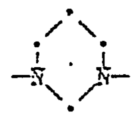 , 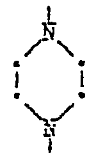 , 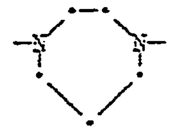 et

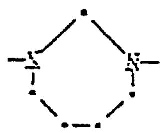 .

6. Composés selon la revendication 1 dans lesquels p est égal à 3 et A représente un radical:

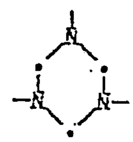

7. Composés selon la revendication 1 dans lesquels:
R′ et R″ représentent chacun, indépendamment l'un de l'autre, un benzyle ou un alkyle en $C_1$–$C_6$,
$R_2$ et $R_3$ représentent chacun l'hydrogène et
A représente, dans le cas où p est égal à 1, un radical $-NHR_4$ dont le symbole $R_4$ désigne un alkyle en $C_1$–$C_{18}$ et, lorsque p est égal à 2, un radical

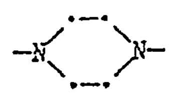

ou un radical

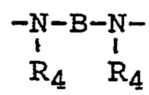

dans lequel B représente un alkylène en $C_1$–$C_{10}$ et $R_4$ a la signification qui lui a été donnée ci-dessus.
8. Composés selon la revendication 1, en l'espèce:
le N-tert-butyl-[(N,N-dibenzylamino-oxy)-3 propionamide],
le N-tert-octyl-[(N,N-dibenzylamino-oxy)-3 propionamide],
le N,N′-diméthyl-N,N′-hexaméthylène-bis-[(N,N-dibenzylamino-oxy)-3 propionamide],
le pipérazinyl-1,4 bis-[(N,N-dibenzylamino-oxy)-3 propionamide], et
le N-tert-octyl-[(N,N-diéthylamino-oxy)-3 propionamide].
9. Composition contenant une matière organique sensible à la dégradation oxydative, thermique et/ou actinique et au moins un composé répondant à la formule I:

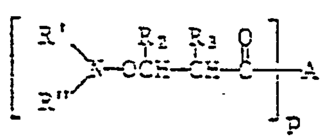 (I)

dans laquelle:
p designe un nombre entier de 1 à 4,
R′ et R″ représentent chacun, indépendamment l'un de l'autre, l'hydrogène, un alkyle en $C_1$–$C_{36}$, un cycloalkyle en $C_5$–$C_{12}$, un aralkyle en $C_7$–$C_9$ ou un aralkyle en $C_7$–$C_9$ porteur d'un alkyle en $C_1$–$C_{36}$,
$R_2$ et $R_3$ représentent chacun, indépendamment l'un de l'autre, l'hydrogène ou un alkyle en $C_1$–$C_{12}$, et
A représente:
– dans le cas où p est égal à 1, un radical $-NHR_4$ dans lequel $R_4$ représente l'hydrogène, un amino, un alkyle en $C_1$–$C_{18}$ ou un radical

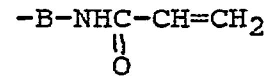

et B représente une liaison directe ou un alkylène en $C_1$–$C_{10}$,

– dans le cas où p est egal à 2, un radical hétérocyclique comportant de 5 à 7 maillons et contenant deux atomes d'azote ayant chacun une liaison libre, ou un radical

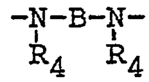

dans lequel B et $R_4$ ont les significations qui leur ont été données ci-dessus,

– dans le cas où p est égal à 3, un radical de formule

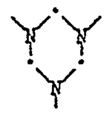

ou un radical de formule:

$$R_5-N-(CH_2)_n-N-(CH_2)_n-N-R_5$$

dans lequel n désigne un nombre entier de 2 à 6 et $R_5$ représente l'hydrogène ou un alkyle en $C_1$–$C_4$, et

– dans le cas où p est égal à 4, un radical de formule

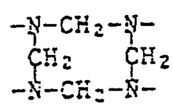

ou un radical de formule:

$$R_5-N-(CH_2)_n-N-(CH_2)_n-N-(CH_2)_n-N-R_5$$

dans lequel $R_5$ et n ont les significations qui leur ont été données ci-dessus.

10. Composition selon la revendication 9 dans laquelle la matière organique est un polymère synthétique.

11. Composition selon la revendication 10 dans laquelle le polymère synthétique est un homopolymère ou un copolymère d'oléfine(s).

12. Composition selon la revendication 9 caractérisée en ce qu'elle contient au moins un additif usuel.

13. Composition selon la revendication 12 dans laquelle l'additif usuel est un sel métallique d'un acide gras supérieur.

14. Composition selon la revendication 12 dans laquelle l'additif usuel est un anti-oxydant phénolique.

15. Composition selon la revendication 12 dans laquelle les additifs usuels sont un sel métallique d'un acide gras supérieur et un anti-oxydant phénolique.

16. Composition selon la revendication 14 dans laquelle l'anti-oxydant phénolique est:
le tétrakis-(di-tert-butyl-3,5 hydroxy-4 hydrocinnamate) de néopentane-tétrayle,
le di-tert-butyl-3,5 hydroxy-4 hydrocinnamate de n-octadécyle,
le triméthyl-1,3,5 tris-(di-tert-butyl-3,5 hydroxy-4 benzyl)2,4,6 benzène,
l'isocyanurate de tris-(di-tert-butyl-3,5 hydroxy-4 benzyle),
le di-tert-butyl-2,6 p-crésol ou
l'éthylidène-2,2′ bis-(di-tert-butyl-4,6 phénol).

17. Application des composés de formule I définis à la revendication 9 pour la stabilisation d'une matière organique contre la dégradation oxydative, thermique et/ou actinique.

## Claims

1. A compound of the formula I,

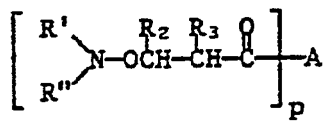 (I)

in which p is an integer from 1 to 4, R′ and R″ independently of one another are hydrogen, $C_1$–$C_{36}$alkyl, $C_5$–$C_{12}$cycloalkyl, $C_7$–$C_9$aralkyl or $C_1$–$C_{35}$alkyl-substituted $C_7$–$C_9$aralkyl, $R_2$ and $R_3$ independently of one another are hydrogen or $C_1$–$C_{12}$alkyl and
if p is 1, A is a group -$NR_4$ in which $R_4$ is hydrogen, amino, $C_1$–$C_{18}$alkyl or

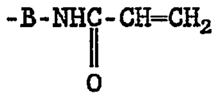

and B is a direct bond or $C_1$–$C_{10}$alkylene,
if p is 2, A is a 5-membered to 7-membered heterocyclic radical which has 2 nitrogen atoms each having a free valency, or is a group

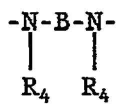

in which B and $R_4$ have the meanings given above,
if p is 3, A is a group of the formula

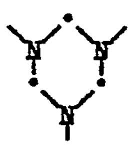 or 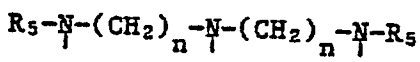

in which n is an integer from 2 to 6 and $R_5$ is hydrogen or $C_1$–$C_4$alkyl, and
if p is 4, A is a group of the formula

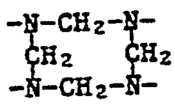 or 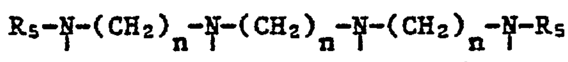

in which $R_5$ and n have the meanings given above, with the exclusion of the compound β-aminoxypropionamide.

2. A compound according to claim 1, in which R′ and R″ independently of one another are hydrogen, $C_1$–$C_{18}$alkyl, cyclopentyl, cyclohexyl, benzyl, α-methylbenzyl or α,α-dimethylbenzyl and $R_2$ and $R_3$ independently of one another are hydrogen or $C_1$–$C_4$alkyl.

3. A compound according to claim 1, in which R′ and R″ are benzyl and $R_2$ and $R_3$ are hydrogen.

4. A compound according to claim 1, in which p is 1, A is a group -$NHR_4$ and $R_4$ is $C_1$–$C_{18}$alkyl.

5. A compound according to claim 1, in which p is 2 and A is

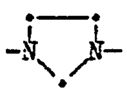 , 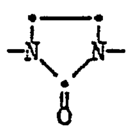 , 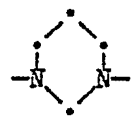 , 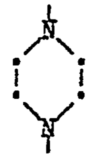 , 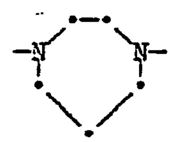 or

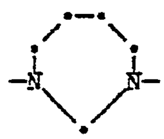

6. A compound according to claim 1, in which p is 3 and A is

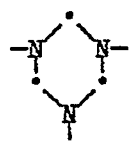

7. A compound according to claim 1, in which R′ and R″ independently of one another are benzyl or $C_1$–$C_6$alkyl, $R_2$ and $R_3$ are hydrogen and, if p is 1, A is a group $-NHR_4$ in which $R_4$ is $C_1$–$C_{18}$alkyl, and, if p is 2, A is a group

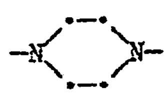 or 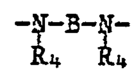

in which B is $C_1$–$C_{10}$alkylene and $R_4$ is as defined above.

8. The compounds N-tert-butyl-[3-(N′,N′-dibenzylaminoxy)propionamide], N-tert-octyl-[3(N′,N′-dibenzylaminoxy)propionamide], N,N′-dimethyl-N,N′-hexamethylene-bis[3-(N″,N″-dibenzylaminoxy)propionamide], 1,4-piperazinyl-bis[3-(N,N-dibenzylaminoxy)propionamide] and N-tertoctyl-[3-(N′N′-diethylaminoxy)propionamide] according to claim 1.

9. A composition containing an organic material sensitive to oxidative, thermal and/or actinic degradation and at least one compound of the formula I,

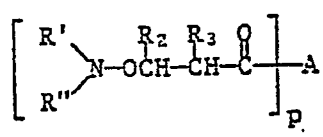 (I)

in which p is an integer from 1 to 4, R′ and R″ independently of one another are hydrogen, $C_1$–$C_{36}$alkyl, $C_5$–$C_{12}$cycloalkyl, $C_7$–$C_9$aralkyl or $C_1$–$C_{36}$alkyl-substituted $C_7$–$C_9$aralkyl, $R_2$ and $R_3$ independently of one another are hydrogen or $C_1$–$C_{12}$alkyl and
if p is 1, A is a group $-NHR_4$ in which $R_4$ is hydrogen, amino, $C_1$–$C_{18}$alkyl or

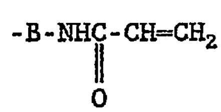

and B is a direct bond or $C_1$–$C_{10}$alkylene,
if p is 2 A is a 5-membered to 7-membered heterocyclic radical which has 2 nitogen atoms each having a free valency, or is a group

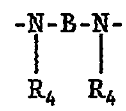

in which B and $R_4$ have the meanings given above,
if p is 3, A is a group of the formula

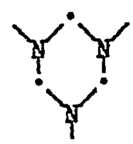

or

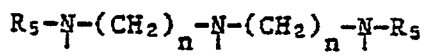

in which n is an integer from 2 to 6 and $R_5$ is hydrogen or $C_1$–$C_4$alkyl, and
if p is 4, A is a group of the formula

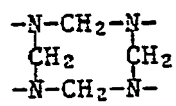

or

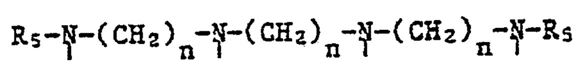

in which $R_5$ and n have the meanings given above.

10. A composition according to claim 9, in which the organic material is a synthetic polymer.

11. A composition according to claim 10, in which the synthetic polymer is a polyolefin homopolymer or copolymer.

12. A composition according to claim 9, which contains at least one conventional additive.

13. A composition according to claim 12, in which the conventional additive is a metal salt of a higher fatty acid.

14. A composition according to claim 12, in which the conventional additive is a phenolic antioxidant.

15. A composition according to claim 12, in which the conventional additives are composed of a metal salt of a higher fatty acid and a phenolic antioxidant.

16. A composition according to claim 14, in which the phenolic antioxidant is neopentanetetrayl tetrakis(3,5-di-tert-butyl-4-hydroxyhydrocinnamate), n-octadecyl 3,5-di-tert-butyl-4-hydroxyhydrocinnamate, 1,3,5-trimethyl-2,4,6-tris(3,5-di-tert-butyl-4-hydroxybenzyl)benzene, 1,3,5-tris(3,5-ditert-butyl-4-hydroxybenzyl) isocyanurate, 2,6-di-tert-butyl-p-cresol or 2,2′-ethylidene-bis(4,6-di-tert-butylphenol).

17. Use of the compounds of the formula I defined in claim 9 for stabilizing organic material against oxidative, thermal and/or actinic degradation.